# EUROPÄISCHE PATENTSCHRIFT

(11) **EP 4 205 686 B1**
(45) Veröffentlichungstag und Bekanntmachung des Hinweises auf die Patenterteilung: **03.06.2026**
(21) Anmeldenummer: 21218116.8
(22) Anmeldetag: 29.12.2021
(51) Int. Cl.: A61B 34/10, A61B 18/02, A61B 90/00, A61B 18/00

(54) **BEREITSTELLEN VON WÄRMELEITFÄHIGKEITSDATEN, WELCHE EINE ANATOMISCHE STRUKTUR BETREFFEN, FÜR EINE KRYOABLATION**
PROVISION OF THERMAL CONDUCTIVITY DATA CONCERNING AN ANATOMICAL STRUCTURE FOR CRYOABLATION
FOURNITURE DE DONNÉES DE CONDUCTIVITÉ THERMIQUE CONCERNANT UNE STRUCTURE ANATOMIQUE POUR LA CRYOABLATION

(43) Veröffentlichungstag der Anmeldung: 05.07.2023
(73) Patentinhaber: Siemens Healthineers AG, 91301 Forchheim (DE)
(72) Erfinder: Hofmann, Bernd, 91058 Erlangen (DE); Lichy, Matthias, 90489 Nürnberg (DE); Schmidt, Bernhard, 90766 Fürth (DE)
(74) Vertreter: Siemens Healthineers Patent Attorneys

(56) Entgegenhaltungen:
- WO-A1-2015/148378
- US-A1- 2010 185 087
- US-A1- 2011 196 385
- POPPENDIEK H.F. ET AL: "Thermal conductivity measurements and predictions for biological fluids and tissues", CRYOBIOLOGY, vol. 3, no. 4, 1 January 1967 (1967-01-01), US, pages 318 - 327, XP055934978, ISSN: 0011-2240, DOI: 10.1016/S0011-2240(67)80005-1
- HUGO TALBOT ET AL: "Interactive Planning of Cryotherapy Using Physically-Based Simulation Interactive Planning of Cryotherapy Using Physics-Based Simulation", 1 February 2014 (2014-02-01), pages 918200, XP055292622, Retrieved from the Internet <URL:https://hal.inria.fr/hal-00918200/file/Talbot_H.pdf> [retrieved on 20160801]

## Beschreibung

Die Erfindung betrifft ein Verfahren zum Bereitstellen von Wärmeleitfähigkeitsdaten, welche eine anatomische Struktur betreffen. Die Erfindung betrifft ferner ein Datenverarbeitungssystem, ein medizinisches Bildgebungssystem und ein medizinisches Kryoablationssystem.

Die Kryoablation spielt eine wesentliche Rolle bei der Behandlung von Tumoren, beispielsweise von Tumoren der Niere, des Pankreas oder des Knochens.

US 2010/185087 A1 offenbart ein Verfahren zur Durchführung eines thermischen Ablationsverfahrens innerhalb eines interessierenden Volumens in einem Patienten.

US 2011/196385 A1 offenbart ein Verfahren zum Bestimmen eines abgetragenen Objektbereichs zum Abtragen eines interessierenden Objekts.

Ähnlich wie bei jeder thermalen Gewebedestruktion stellen die Visualisierung der Ablationszone und die Veränderung der Temperatur im behandelten und angrenzenden Gewebe auch bei der Kryoablation einen entscheidenden Faktor hinsichtlich des Therapieerfolges dar. Wichtig ist dabei, eine komplette Ablation des Tumorgewebes zu bewirken ohne dabei relevante benachbarte Strukturen zu zerstören.

Eine prä-interventionelle Bestimmung der thermischen Gewebeleitfähigkeit ist essentiell zur Planung der Sondenlokalisation und der Abschätzung von möglichen Schäden im gesunden Gewebe. Beide Faktoren gehen in die Interventionsplanung ein. Während der Ablation muss eine möglichst exakte Darstellung der vereisten Zone erfolgen. Ähnlich wie bei anderen thermischen Ablationen kann es hierbei durch thermische Brücken (große Gefäße) zu unzureichenden Ergebnissen kommen. Dabei ist insbesondere die zeitliche Veränderung der Ablationszone von Relevanz. Allgemein stellen Bildartefakte durch die eingebrachten Sonden ein Problem bei der Durchführung von thermischen Ablationen dar.

Zur Planung und Kontrolle der Kryoablation kann ein medizinisches Bildgebungssystem, beispielsweise basierend auf Ultraschallbildgebung, Magnetresonanztomographie (MRT) oder Computertomographie (CT) verwendet werden.

Im Ultraschallbild kommt es aufgrund der Vereisung zu einer Reflexion und damit Auslöschung der Gewebeinformationen. Der Fettgehalt des Gewebes beeinflusst die visuelle Darstellung der Organtextur. Thermische Brücken in Form von Gefäßen können beurteilt werden (Doppler, CEUS). Problematisch ist hierbei, dass es sich um rein qualitative Informationen handelt und dass sowohl die Sonde als auch die Vereisung zu Veränderungen der Bildinformation führen und somit eine genaue Darstellung der Ablationszone erschweren.

Bei der MRT können mittels spezieller Sequenzen die Temperaturveränderungen im Gewebe dargestellt werden. Es ist auch möglich, eine semiquantitative Fettanalyse durchzuführen. Diese Messsequenzen sind jedoch stark artefaktanfällig. Darüber hinaus kommt es aufgrund der Vereisung zu Suszeptibilitätsartefakten und damit zu einer unzureichenden Beurteilung der genauen Ausdehnung des zerstörten Gewebes.

Die Erfindung hat die Aufgabe, eine Alternative zu einer herkömmlichen Planung und Überwachung einer Kryoablation zu ermöglichen. Jeder Gegenstand eines unabhängigen Anspruchs löst diese Aufgabe. In den abhängigen Ansprüchen sind weitere vorteilhafte Aspekte der Erfindung berücksichtigt.

Die Erfindung betrifft ein Verfahren zum Bereitstellen von Wärmeleitfähigkeitsdaten, welche eine anatomische Struktur betreffen, das Verfahren umfassend:
- ein Empfangen von ersten spektralen Computertomographiedaten, welche die anatomische Struktur betreffen, wobei die anatomische Struktur eine Niere ist und eine flüssigkeitsführende Teilstruktur in Form eines Nierenkelchsystems aufweist,
- ein Berechnen einer Fettkarte der anatomischen Struktur und einer Wasserkarte der anatomischen Struktur basierend auf den ersten spektralen Computertomographiedaten,
- ein Berechnen der Wärmeleitfähigkeitsdaten, welche die anatomische Struktur betreffen, basierend auf der Fettkarte und der Wasserkarte,
- ein Bereitstellen der Wärmeleitfähigkeitsdaten.

Hiermit wird ferner ein Verfahren zum Bereitstellen von Wärmeleitfähigkeitsdaten, welche eine anatomische Struktur betreffen, offenbart, das Verfahren umfassend:
- ein Empfangen von ersten spektralen Computertomographiedaten, welche die anatomische Struktur betreffen,
- ein Berechnen der Wärmeleitfähigkeitsdaten, welche die anatomische Struktur betreffen, basierend auf den ersten spektralen Computertomographiedaten,
- ein Bereitstellen der Wärmeleitfähigkeitsdaten.

Spektrale Computertomographiedaten, insbesondere die ersten spektralen Computertomographiedaten, können beispielsweise basierend auf photonenzählender Computertomographie und/oder Dual-Energy-Computertomographie, insbesondere Dual-Source-Computertomographie, aufgenommen sein.

Die anatomische Struktur kann beispielsweise ein Organ, insbesondere eine Niere, ein Pankreas oder ein Knochen, sein. Die anatomische Struktur kann beispielsweise das Organ und ein an das Organ angrenzendes Gewebe aufweisen. Die Fettkarte der anatomischen Struktur und die Wasserkarte der anatomischen Struktur können beispielsweise basierend auf einer Materialzerlegung und/oder einer Multikompartmentsegmentierung berechnet werden. Beispielsweise können die Fettkarte der anatomischen Struktur und die Wasserkarte der anatomischen Struktur berechnet werden, indem ein Fettkompartiment und ein Wasserkompartiment basierend auf den ersten spektralen Computertomographiedaten segmentiert werden, insbesondere gleichzeitig segmentiert werden.

Die Wärmeleitfähigkeitsdaten können beispielsweise eine Wärmeleitfähigkeitskarte der anatomischen Struktur umfassen. Die Wärmeleitfähigkeitsdaten können beispielsweise eine thermale Gewebeleitfähigkeit betreffen und/oder zur Planung und/oder Überwachung einer Kryoablation verwendet werden. Die thermale Gewebeleitfähigkeit wird im Wesentlichen durch das relative Verhältnis von Fett und Wasser bestimmt. Einem Bildpunkt mit einem relativ hohen Wassergehalt kann beispielsweise eine höhere Wärmeleitfähigkeit zugeordnet werden als einem Bildpunkt mit einem relativ hohen Fettgehalt. Die Fettkarte und die Wasserkarte können beispielsweise zur Planung einer Kryotherapie einer zu behandelnden Region der anatomischen Struktur, beispielsweise zur Berechnung einer Ausdehnung einer Kryoablation und/oder zur Positionierung einer Kryoablationssonde, verwendet werden.

Erfindungsgemäß ist vorgesehen, dass eine Darstellung der flüssigkeitsführenden Teilstruktur der anatomischen Struktur basierend auf der Fettkarte der anatomischen Struktur und/oder der Wasserkarte der anatomischen Struktur generiert wird, wobei die Wärmeleitfähigkeitsdaten basierend auf der Darstellung der flüssigkeitsführenden Teilstruktur der anatomischen Struktur berechnet werden.

Die flüssigkeitsführende Teilstruktur der anatomischen Struktur kann beispielsweise eine thermische Brücke bilden. Über die thermische Brücke kann beispielsweise ein Wärmeeintrag in die Kryoablationszone erfolgen, der dem Wärmeentzug, welcher mittels der Kryoablationssonde erfolgt, entgegenwirkt. Die Darstellung der flüssigkeitsführenden Teilstruktur kann beispielsweise generiert werden, indem sie basierend auf einem erhöhten Wassergehalt der flüssigkeitsführenden Teilstruktur berechnet wird.

Eine Ausführungsform sieht vor, dass die anatomische Struktur eine zu behandelnde Region aufweist, wobei basierend auf den Wärmeleitfähigkeitsdaten eine Darstellung einer Vereisungszone für eine Kryoablation der zu behandelnden Region berechnet wird.

Die zu behandelnde Region kann beispielsweise ein Tumor sein. Die Vereisungszone kann insbesondere als Soll-Ablationszone verstanden werden, deren Vereisung eine erfolgreiche Behandlung der zu behandelnden Region bewirkt, ohne dabei relevante benachbarte Strukturen zu zerstören. Die Darstellung der Vereisungszone kann ferner basierend auf einer Darstellung der zu behandelnden Region und/oder einer zu verschonenden Region berechnet werden. Insbesondere kann eine Grenzzone von Interesse, beispielsweise in Form eines Übergangs zwischen dem Cortex und dem perirenalen Gewebe, bei der Berechnung der Darstellung der Vereisungszone berücksichtigt werden.

Eine Ausführungsform sieht vor, dass basierend auf den Wärmeleitfähigkeitsdaten für jeden Zeitpunkt einer Mehrzahl von aufeinanderfolgenden Zeitpunkten jeweils eine Darstellung der Vereisungszone, welche diesen Zeitpunkt betrifft, berechnet wird. Daraus können beispielsweise Informationen über eine Vereisungsgeschwindigkeit erzeugt werden.

Eine Ausführungsform sieht vor, dass basierend auf den Wärmeleitfähigkeitsdaten eine Position für eine Kryoablationssonde berechnet wird, insbesondere für die Kryoablation der zu behandelnden Region berechnet wird. Die Position der Kryoablationssonde kann beispielsweise auf die anatomische Struktur und/oder auf ein Koordinatensystem eines Kryoablationssystems bezogen sein. Weiterhin kann vorgesehen sein, dass für jede Kryoablationssonde einer Mehrzahl von Kryoablationssonden basierend auf den Wärmeleitfähigkeitsdaten eine Position für diese Kryoablationssonde berechnet wird, insbesondere für die Kryoablation der zu behandelnden Region berechnet wird.

Durch den Wärmeentzug mittels der Kryoablationssonde kann insbesondere eine Vereisung und damit eine Dichteänderung bewirkt werden. Ein Betrag der Dichteänderung entspricht dabei einem Grad der Vereisung. Die Dichteänderungskarte kann insbesondere eine Röntgendichte betreffen und/oder jedem Bildpunkt einer Mehrzahl von Bildpunkten einen CT-Wert, beispielsweise auf einer Hounsfield-Skala, zuordnen.

Mit Hilfe der spektralen Computertomographie kann die Dichteänderung insbesondere unabhängig von einer Beurteilung des Wassergehalts und des Fettgehalts bestimmt werden, da es zu keiner vollständigen Auslöschung der Röntgenstrahlung im Bereich der Vereisung kommt.

Eine Ausführungsform sieht vor, dass basierend auf der Dichteänderungskarte eine Vereisungskarte der zu behandelnden Region berechnet wird.

Weiterhin kann vorgesehen sein, dass die Vereisungskarte ferner basierend auf den Wärmeleitfähigkeitsdaten berechnet wird und/oder dass die Vereisungskarte ferner basierend auf der Fettkarte der anatomischen Struktur und/oder der Wasserkarte der anatomischen Struktur berechnet wird. Die Vereisungskarte kann insbesondere jedem Bildpunkt einer Mehrzahl von Bildpunkten einen Grad der Vereisung zuordnen. Bildpunkte können beispielsweise 2D-Bildpunkte (Pixel) oder 3D-Bildpunkte (Voxel, Volumenelemente) sein.

Weiterhin kann vorgesehen sein, dass die basierend auf den Wärmeleitfähigkeitsdaten berechnete Darstellung der Vereisungszone der Dichteänderungskarte und/oder der Vereisungskarte überlagert dargestellt wird.

Eine Ausführungsform sieht vor, dass ein Betriebsparameter der Kryoablationssonde berechnet wird, indem ein Regelungsalgorithmus auf die Darstellung der Vereisungszone als Führungsgröße und auf die Vereisungskarte als Regelgröße angewendet wird. Damit kann beispielsweise das Erreichen einer optimalen Vereisung kontrolliert werden und/oder eine verbesserte Anpassung an eine zu verschonende Region erfolgen, um eine möglichst vollständige Ablation zu erreichen. Der Betriebsparameter der Kryoablationssonde kann beispielsweise eine Temperatur der Kryoablationssonde und/oder eine Wärmeentzugsleistung der Kryoablationssonde betreffen. Insbesondere kann die Kryoablationssonde basierend auf dem Betriebsparameter der Kryoablationssonde gesteuert werden.

Ein zeitlicher Verlauf der Vereisung kann beispielsweise basierend auf einer Vektoranalyse über die Mehrzahl von Vereisungskarten analysiert und/oder mit den Wärmeleitfähigkeitsdaten und/oder der Darstellung der Vereisungszone, insbesondere für jeden Zeitpunkt der Mehrzahl von aufeinanderfolgenden Zeitpunkten, für die jeweils eine Darstellung der Vereisungszone berechnet wurde, korreliert werden, insbesondere um ein Ausmaß der Vereisung beurteilen zu können, beispielsweise hinsichtlich einer Übereinstimmung mit einem geplanten Verlauf der Kryoablation beurteilen zu können.

Die ersten spektralen Computertomographiedaten können insbesondere aufgenommen werden, ohne dafür die anatomische Struktur mit einem Kontrastmittel zu versehen.

Sollte während der Kryoablation, beispielsweise bei Verdacht auf eine akute Blutung, eine Kontrastmittel-gestütztes Computertomographie notwendig werden, ist unter Bezug auf die vorhandenen Dichtekarten eine spektrale Segmentierung von Jod auch innerhalb der Ablationszone möglich, beispielsweise um eine Jodkarte der anatomischen Struktur zu erzeugen.

Residuelle Einblutungen und größere Nekroseareale können ebenfalls wie oben beschrieben visualisiert und/oder während der Kryoablation kontrolliert werden. Über eine Veränderung des Wassergehalts und/oder der Dichte kann ebenfalls eine Kontrolle der Vereisung erfolgen.

Die Erfindung betrifft ferner ein Datenverarbeitungssystem zum Bereitstellen von Wärmeleitfähigkeitsdaten, welche eine anatomische Struktur betreffen, aufweisend eine Datenschnittstelle und einen Prozessor, wobei das Datenverarbeitungssystem dazu eingerichtet ist, ein erfindungsgemäßes Verfahren auszuführen.

Die Erfindung betrifft ferner ein medizinisches Bildgebungssystem, aufweisend das erfindungsgemäße Datenverarbeitungssystem und ein Computertomographiegerät zur Aufnahme der ersten spektralen Computertomographiedaten.

Das Computertomographiegerät kann beispielsweise zur Aufnahme von spektralen Computertomographiedaten, insbesondere der ersten spektralen Computertomographiedaten, basierend auf photonenzählender Computertomographie und/oder Dual-Energy-Computertomographie, insbesondere Dual-Source-Computertomographie, eingerichtet sein.

Die Erfindung betrifft ferner ein medizinisches Kryoablationssystem, aufweisend das medizinische Bildgebungssystem und eine Kryoablationssonde.

Somit kann das Bereitstellen von Wärmeleitfähigkeitsdaten insbesondere eine funktionelle Therapieplanung ermöglichen. Dabei ist insbesondere im Vergleich zur Bildfusion in der Magnetresonanztomographie der Aufwand für die vor Beginn der Ablation durchzuführende Bildgebung verringert. Auch während der Ablation können die Vorteile der Computertomographie in Bezug auf 3D-Bildgebung und Schnelligkeit sowie die Verwendung von Metallteilen im Untersuchungsbereich genutzt werden.

Außerdem ist ein Therapiemonitoring der Kryoablationszone unter Vereisung möglich. Damit kann eine Kryoablation in Bezug auf eine genaue und vollständige Abdeckung der zu behandelnden Region sowie den Grad der Vereisung beurteilt und dokumentiert, insbesondere quantitativ beurteilt und dokumentiert werden.

Das Verfahren zum Bereitstellen von Wärmeleitfähigkeitsdaten kann insbesondere ein computerimplementiertes Verfahren sein.

Die Erfindung betrifft ferner ein Computerprogrammprodukt, umfassend Anweisungen, die, wenn die Anweisungen von einem Computer ausgeführt werden, den Computer veranlassen, das erfindungsgemäße Verfahren auszuführen.

Das Computerprogrammprodukt kann beispielsweise ein Computerprogramm sein oder neben dem Computerprogramm mindestens einen zusätzlichen Bestandteil umfassen. Der mindestens eine zusätzliche Bestandteil des Computerprogrammprodukts kann als Hardware und/oder als Software ausgebildet sein.

Das Computerprogrammprodukt kann beispielsweise ein Speichermedium, auf dem zumindest ein Teil des Computerprogrammprodukts gespeichert ist, und/oder ein Schlüssel zur Authentifizierung eines Benutzers des Computerprogrammprodukts, insbesondere in Form eines Dongles, aufweisen. Das Computerprogrammprodukt und/oder das Computerprogramm kann beispielsweise ein Cloud-Anwendungs-Programm aufweisen, welches zum Verteilen der Anweisungen auf verschiedene Verarbeitungseinheiten, insbesondere verschiedene Computer, eines Cloud-Computing-Systems ausgebildet ist, wobei jede der Verarbeitungseinheiten zum Ausführen einer oder mehrerer der Anweisungen ausgebildet ist.

Die Erfindung betrifft ferner ein computerlesbares Speichermedium, umfassend Anweisungen, die, wenn die Anweisungen von einem Computer ausgeführt werden, den Computer veranlassen, das erfindungsgemäße Verfahren auszuführen.

Auf dem computerlesbaren Speichermedium kann beispielsweise das Computerprogrammprodukt nach einer der Ausführungsformen, die in dieser Anmeldung offenbart sind, und/oder das Computerprogramm nach einer der Ausführungsformen, die in dieser Anmeldung offenbart sind, gespeichert sein. Das computerlesbare Speichermedium kann beispielsweise ein Memorystick, eine Festplatte oder ein sonstiger Datenträger sein, der insbesondere lösbar mit einem Computer verbunden oder fest in einen Computer integriert sein kann. Das computerlesbare Speichermedium kann beispielsweise einen Bereich eines Speichersystems bilden, wobei das Datenverarbeitungssystem mittels der Datenschnittstelle mit dem Speichersystem verbunden ist.

Das Datenverarbeitungssystem kann beispielsweise eine oder mehrere Komponenten in Form von Hardware und/oder eine oder mehrere Komponenten in Form von Software aufweisen. Das Datenverarbeitungssystem kann beispielsweise zumindest teilweise von einem Cloud-Computing-System gebildet sein. Das Datenverarbeitungssystem kann beispielsweise ein Cloud-Computing-System, ein Computernetzwerk, ein Computer, ein Tabletcomputer, ein Smartphone oder ähnliches oder eine Kombination davon sein und/oder aufweisen.

Die Hardware kann beispielsweise mit einer Software zusammenwirken und/oder mittels einer Software konfigurierbar sein. Die Software kann beispielsweise mittels der Hardware ausgeführt werden. Bei der Hardware kann es sich beispielsweise um ein Speichersystem, ein FPGA-System (Field-programmable gate array), ein ASIC-System (Application-specific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen.

Die Schritte des Verfahrens können beispielsweise in dem Prozessor des Datenverarbeitungssystems, insbesondere in Form von Berechnungen, ausgeführt werden. Ein Berechnen, beispielsweise das Berechnen der Wärmeleitfähigkeitsdaten und/oder das Berechnen der Darstellung der Vereisungszone, kann insbesondere erfolgen, indem ein Algorithmus, beispielsweise ein trainierter Maschinenlernalgorithmus, auf diejenigen Daten, auf denen das Berechnen basiert, angewendet wird.

Ein Datentransfer zwischen Komponenten des medizinischen Bildgebungssystems und/oder des medizinischen Kryoablationssystems kann beispielsweise jeweils mittels einer geeigneten Datentransfer-Schnittstelle erfolgen. Die Datentransfer-Schnittstelle zum Datentransfer an und/oder von einer Komponente des medizinischen Bildgebungssystems und/oder des medizinischen Kryoablationssystems kann zumindest teilweise in Form von Software und/oder zumindest teilweise in Form von Hardware realisiert sein. Die Datentransfer-Schnittstelle kann beispielsweise zum Abspeichern von Daten in und/oder zum Einlesen von Daten aus einem Bereich eines Speichersystems ausgebildet sein, wobei auf diesen Bereich des Speichersystems eine oder mehrere Komponenten des medizinischen Bildgebungssystems und/oder des medizinischen Kryoablationssystems zugreifen können.

Daten, insbesondere die ersten Computertomographiedaten, können beispielsweise empfangen werden, indem ein Signal, welches die Daten trägt, empfangen wird und/oder indem die Daten eingelesen werden, insbesondere aus einem computerlesbaren Speichermedium eingelesen werden. Daten, insbesondere die Wärmeleitfähigkeitsdaten, die Darstellung der Vereisungszone, die Fettkarte, die Wasserkarte, die Dichteänderungskarte, die Vereisungskarte und der Betriebsparameter der Kryoablationssonde, können beispielsweise bereitgestellt werden, indem ein Signal, welches die Daten trägt, übertragen wird und/oder indem die Daten in ein computerlesbares Speichermedium geschrieben werden und/oder indem die Daten auf einem Bildschirm angezeigt werden.

Die Wärmeleitfähigkeitsdaten, die Darstellung der Vereisungszone, die Fettkarte, die Wasserkarte, die Dichteänderungskarte und die Vereisungskarte können jeweils insbesondere in Form von zweidimensionalen Bilddaten oder dreidimensionalen Bilddaten strukturiert sein.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung des unbestimmten Artikels "ein" bzw. "eine" schließt nicht aus, dass das betroffene Merkmal auch mehrfach vorhanden sein kann. Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal berechnet (alternativ: ermittelt, generiert etc.) wird, nicht aus, dass das erste Merkmal ferner basierend auf einem dritten Merkmal berechnet (alternativ: ermittelt, generiert etc.) werden kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Die Fig. 1 zeigt eine anatomische Struktur in einem ersten Zustand der anatomischen Struktur.

Die Fig. 2 zeigt die anatomische Struktur in einem zweiten Zustand der anatomischen Struktur.

Die Fig. 3 zeigt ein Ablaufdiagramm eines Verfahrens zum Bereitstellen von Wärmeleitfähigkeitsdaten, welche eine anatomische Struktur N betreffen.

Die Fig. 4 zeigt ein medizinisches Kryoablationssystem.

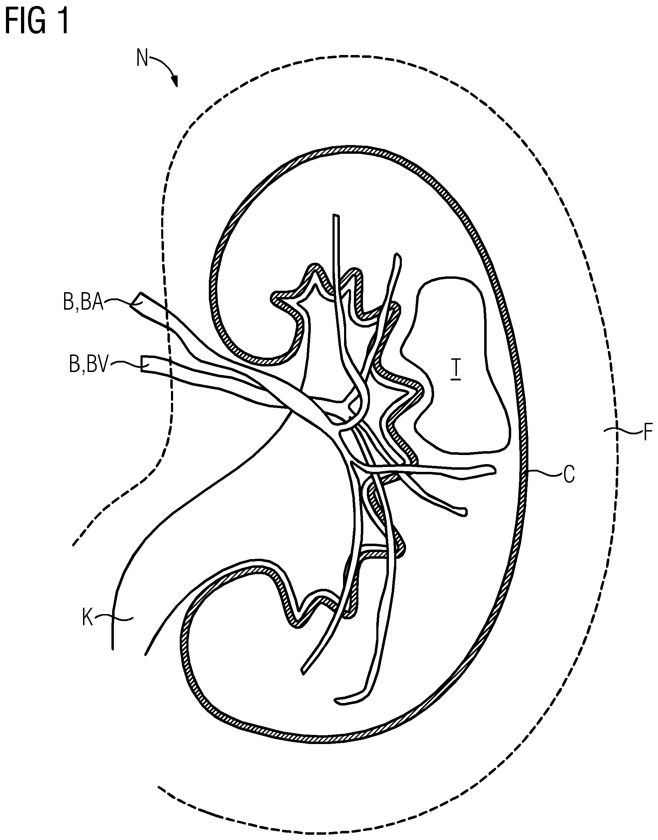

Die Fig. 1 zeigt die anatomische Struktur N in einem ersten Zustand der anatomischen Struktur N, in dem keine Kryoablationssonde in die anatomische Struktur N eingeführt ist. Die anatomische Struktur N ist beispielhaft als Niere mit der Nierenkapsel C und dem perirenalen Gewebe F dargestellt. Die anatomische Struktur N weist eine flüssigkeitsführende Teilstruktur in Form der Blutgefäße B, insbesondere in Form der Nierenarterie BA und der Nierenvene BV, und eine flüssigkeitsführende Teilstruktur in Form des Nierenkelchsystems K auf. Die anatomische Struktur N weist ferner die zu behandelnde Region T in Form eines Tumors auf.

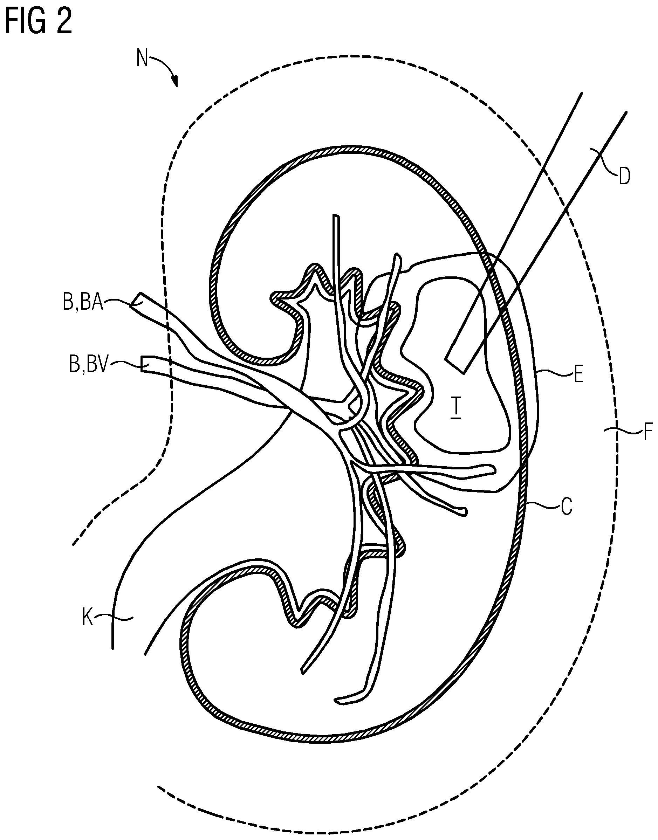

Die Fig. 2 zeigt die anatomische Struktur N in einem zweiten Zustand der anatomischen Struktur N, in dem die Kryoablationssonde D in die anatomische Struktur N eingeführt ist. Außerdem ist die Vereisungszone E für die Kryoablation der zu behandelnden Region T dargestellt.

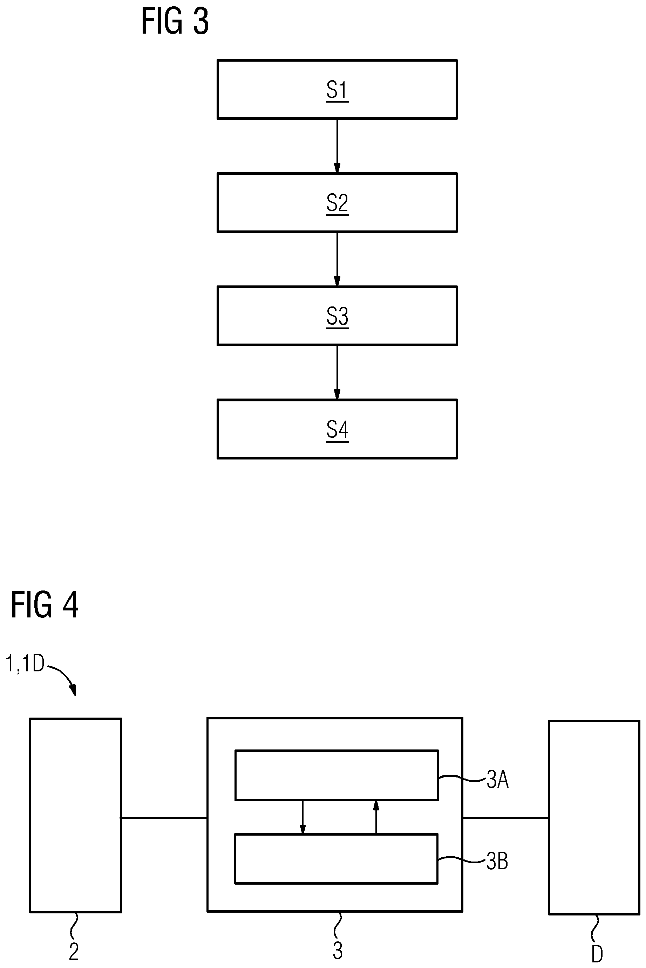

Die Fig. 3 zeigt ein Ablaufdiagramm eines Verfahrens zum Bereitstellen von Wärmeleitfähigkeitsdaten, welche eine anatomische Struktur N betreffen, das Verfahren umfassend:
- ein Empfangen S1 von ersten spektralen Computertomographiedaten, welche die anatomische Struktur N betreffen,
- ein Berechnen S2 einer Fettkarte der anatomischen Struktur N und einer Wasserkarte der anatomischen Struktur N basierend auf den ersten spektralen Computertomographiedaten,
- ein Berechnen S3 der Wärmeleitfähigkeitsdaten, welche die anatomische Struktur N betreffen, basierend auf der Fettkarte und der Wasserkarte,
- ein Bereitstellen S4 der Wärmeleitfähigkeitsdaten.

Die Fig. 4 zeigt das medizinische Kryoablationssystem 1D, aufweisend das medizinische Bildgebungssystem 1 und die Kryoablationssonde D. Das medizinische Bildgebungssystem 1 weist das Datenverarbeitungssystem 3 und das Computertomographiegerät 2 zur Aufnahme der ersten spektralen Computertomographiedaten auf. Das Datenverarbeitungssystem 3 zum Bereitstellen von Wärmeleitfähigkeitsdaten weist die Datenschnittstelle 3A und den Prozessor 3B auf, wobei das Datenverarbeitungssystem 3 dazu eingerichtet ist, das in der Fig. 3 gezeigte Verfahren auszuführen. Das Datenverarbeitungssystem 3 ist mit dem Computertomographiegerät 2 verbunden, insbesondere kabelbasiert oder kabellos verbunden, um die spektralen Computertomographiedaten von dem Computertomographiegerät zu empfangen. Das Datenverarbeitungssystem 3 ist mit der Kryoablationssonde D verbunden, insbesondere kabelbasiert oder kabellos verbunden, um den Betriebsparameter der Kryoablationssonde D an die Kryoablationssonde D bereitzustellen.

## Patentansprüche

1. Verfahren zum Bereitstellen von Wärmeleitfähigkeitsdaten, welche eine anatomische Struktur (N) betreffen, das Verfahren umfassend:
- ein Empfangen (S1) von ersten spektralen Computertomographiedaten, welche die anatomische Struktur (N) betreffen, wobei die anatomische Struktur (N) eine Niere ist und eine flüssigkeitsführende Teilstruktur in Form eines Nierenkelchsystems aufweist,
- ein Berechnen (S2) einer Fettkarte der anatomischen Struktur (N) und einer Wasserkarte der anatomischen Struktur (N) basierend auf den ersten spektralen Computertomographiedaten,
- ein Berechnen (S3) der Wärmeleitfähigkeitsdaten, welche die anatomische Struktur (N) betreffen, basierend auf der Fettkarte und der Wasserkarte, wobei eine Darstellung der flüssigkeitsführenden Teilstruktur der anatomischen Struktur (N) basierend auf der Fettkarte der anatomischen Struktur (N) und/oder der Wasserkarte der anatomischen Struktur (N) generiert wird, wobei die Wärmeleitfähigkeitsdaten basierend auf der Darstellung der flüssigkeitsführenden Teilstruktur der anatomischen Struktur (N) berechnet werden,
- ein Bereitstellen (S4) der Wärmeleitfähigkeitsdaten.

2. Verfahren nach Anspruch 1,
- wobei die anatomische Struktur (N) eine zu behandelnde Region (T) aufweist,
- wobei basierend auf den Wärmeleitfähigkeitsdaten eine Darstellung einer Vereisungszone (E) für eine Kryoablation der zu behandelnden Region (T) berechnet wird.

3. Verfahren nach Anspruch 2
- wobei basierend auf den Wärmeleitfähigkeitsdaten für jeden Zeitpunkt einer Mehrzahl von aufeinanderfolgenden Zeitpunkten jeweils eine Darstellung der Vereisungszone (E), welche diesen Zeitpunkt betrifft, berechnet wird.

4. Verfahren nach einem der Ansprüche 1 bis 3,
- wobei basierend auf den Wärmeleitfähigkeitsdaten eine Position für eine Kryoablationssonde (D) berechnet wird.

5. Datenverarbeitungssystem (3) zum Bereitstellen von Wärmeleitfähigkeitsdaten, aufweisend eine Datenschnittstelle (3A) und einen Prozessor (3B), wobei das Datenverarbeitungssystem (3) dazu eingerichtet ist, ein Verfahren nach einem der Ansprüche 1 bis 4 auszuführen.

6. Medizinisches Bildgebungssystem (1), aufweisend das Datenverarbeitungssystem (3) nach Anspruch 5 und ein Computertomographiegerät (2) zur Aufnahme der ersten spektralen Computertomographiedaten.

7. Medizinisches Kryoablationssystem (1D), aufweisend das medizinische Bildgebungssystem (1) nach Anspruch 6 und eine Kryoablationssonde (D).

8. Computerprogrammprodukt, umfassend Anweisungen, die, wenn die Anweisungen von einem Computer ausgeführt werden, den Computer veranlassen, das Verfahren nach einem der Ansprüche 1 bis 4 auszuführen.

9. Computerlesbares Speichermedium, umfassend Anweisungen, die, wenn die Anweisungen von einem Computer ausgeführt werden, den Computer veranlassen, das Verfahren nach einem der Ansprüche 1 bis 4 auszuführen.

## Claims

1. Method for providing thermal conductivity data relating to an anatomical structure (N), the method comprising:
- receiving (S1) first spectral computed tomography data relating to the anatomical structure (N), wherein the anatomical structure (N) is a kidney and has a fluid-guiding substructure in the form of a renal calyx system,
- calculating (S2) a fat map of the anatomical structure (N) and a water map of the anatomical structure (N) on the basis of the first spectral computed tomography data,
- calculating (S3) the thermal conductivity data relating to the anatomical structure (N) on the basis of the fat map and the water map, wherein a representation of a fluid-guiding substructure of the anatomical structure (N) is generated on the basis of the fat map of the anatomical structure (N) and/or the water map of the anatomical structure (N), wherein the thermal conductivity data is calculated on the basis of the representation of the fluid-guiding substructure of the anatomical structure (N),
- providing (S4) the thermal conductivity data.

2. Method according to claim 1,
- wherein the anatomical structure (N) has a region to be treated (T),
- wherein a representation of a freezing zone (E) for a cryoablation of the region to be treated (T) is calculated on the basis of the thermal conductivity data.

3. Method according to claim 2,
- wherein, for each time point of a plurality of consecutive time points, a representation of the freezing zone (E) relating to this time point is calculated in each case on the basis of the thermal conductivity data.

4. Method according to one of claims 1 to 3,
- wherein a position for a cryoablation probe (D) is calculated on the basis of the thermal conductivity data.

5. Data processing system (3) for providing thermal conductivity data, having a data interface (3A) and a processor (3B), wherein the data processing system (3) is configured to carry out a method according to one of claims 1 to 4.

6. Medical imaging system (1), having the data processing system (3) according to claim 5 and a computed tomography device (2) for recording the first spectral computed tomography data.

7. Medical cryoablation system (1D), having the medical imaging system (1) according to claim 6 and a cryoablation probe (D).

8. Computer program product, comprising instructions which, when the instructions are executed by a computer, cause the computer to carry out the method according to one of claims 1 to 4.

9. Computer-readable storage medium, comprising instructions which, when the instructions are executed by a computer, cause the computer to carry out the method according to one of claims 1 to 4.

## Revendications

1. Procédé pour disposer de données de conductibilité thermique, qui concerne une structure (N) anatomique, le procédé comprenant :
- une réception (S1) de premières données spectrales de tomodensitométrie assistée par ordinateur, qui concernent la structure (N) anatomique, dans lequel la structure (N) anatomique est un rein et une structure partielle conduisant du liquide à la forme d'un système des calices du rein,
- un calcul (S2) d'une carte de graisse de la structure (N) anatomique et d'une carte d'eau de la structure (N) anatomique reposant sur les premières données spectrales de tomodensitométrie assistée par ordinateur,
- un calcul (S3) des données de conductibilité thermique, qui concernent la structure (N) anatomique, reposant sur la carte de graisse et la carte d'eau, dans lequel on crée une représentation de la structure partielle conduisant du liquide de la structure (N) anatomique sur la base de la carte de graisse de la structure (N) anatomique et/ou de la carte d'eau de la structure (N) anatomique, dans lequel on calcule les données de conductibilité thermique sur la base de la représentation de la structure partielle conduisant du liquide de la structure (N) anatomique,
- on met (S4) à disposition les données de conductibilité thermique.

2. Procédé suivant la revendication 1,
- dans lequel la structure (N) anatomique a une région (T) à traiter,
- dans lequel, sur la base des données de conductibilité thermique, on calcule une représentation d'une zone (E) de congélation pour une cryoablation de la région (T) à traiter.

3. Procédé suivant la revendication 2,
- dans lequel, sur la base des données de conductibilité thermique, on calcule, pour chaque instant d'une pluralité d'instants successifs, respectivement, une représentation de la zone (E) de congélation, qui concerne cet instant.

4. Procédé suivant l'une des revendications 1 à 3,
- dans lequel, sur la base des données de conductibilité thermique, on calcule une position d'une sonde (D) de cryoablation.

5. Système (3) de traitement de données, pour disposer de données de conductibilité thermique, comportant une interface (3A) de données et un processeur (3B), dans lequel le système (3) de traitement de données est agencé pour exécuter un procédé suivant l'une des revendications 1 à 4.

6. Système (1) d'imagerie médicale, comportant le système (3) de traitement de données de la revendication 5 et un appareil (2) de tomodensitométrie assisté par ordinateur pour l'enregistrement des premières données spectrales de tomodensitométrie assistée par ordinateur.

7. Système (1D) de cryoablation médical, comportant le système (1) d'imagerie médicale suivant la revendication 6 et une sonde (D) de cryoablation.

8. Produit de programme d'ordinateur, comprenant des instructions, qui, lorsque les instructions sont exécutées par un ordinateur, font que l'ordinateur exécute le procédé suivant l'une des revendications 1 à 4.

9. Support de mémoire déchiffrable par ordinateur, comprenant des instructions, qui, lorsque les instructions sont exécutées par ordinateur, font que l'ordinateur exécute le procédé suivant l'une des revendications 1 à 4.
